(19) Europäisches Patentamt
European Patent Office
Office européen des brevets

(11) **EP 2 825 926 B1**

(12) **EUROPÄISCHE PATENTSCHRIFT**

(45) Veröffentlichungstag und Bekanntmachung des Hinweises auf die Patenterteilung:
**25.07.2018 Patentblatt 2018/30**

(21) Anmeldenummer: **13708444.8**

(22) Anmeldetag: **11.03.2013**

(51) Int Cl.:
*G05D 11/13* (2006.01)    *G05D 24/02* (2006.01)
*C07C 263/20* (2006.01)

(86) Internationale Anmeldenummer:
**PCT/EP2013/054843**

(87) Internationale Veröffentlichungsnummer:
**WO 2013/135614 (19.09.2013 Gazette 2013/38)**

(54) **VERFAHREN ZUR REGELUNG DER VISKOSITÄT EINES MINDESTENS ZWEI KOMPONENTEN MIT UNTERSCHIEDLICHER VISKOSITÄT ENTHALTENDEN GEMISCHS**

METHOD FOR CONTROLLING THE VISCOSITY OF A MIXTURE CONTAINING AT LEAST TWO COMPONENTS HAVING DIFFERENT VISCOSITIES

PROCÉDÉ DE RÉGULATION DE LA VISCOSITÉ D'UN MÉLANGE CONTENANT AU MOINS DEUX COMPOSANTS DE VISCOSITÉS DIFFÉRENTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.03.2012 EP 12159139**

(43) Veröffentlichungstag der Anmeldung:
**21.01.2015 Patentblatt 2015/04**

(73) Patentinhaber: **BASF SE**
**67056 Ludwigshafen am Rhein (DE)**

(72) Erfinder:
• **THIELE, Kai**
**B-2040 Antwerpen 4 (BE)**
• **DUCHEYNE, Wouter**
**B-2018 Antwerpen (BE)**
• **JANSEN, Bart**
**B-2920 Kalmthout (BE)**

(74) Vertreter: **BASF IP Association**
**BASF SE**
**G-FLP-C006**
**67056 Ludwigshafen (DE)**

(56) Entgegenhaltungen:
EP-A2- 1 932 828      FR-A1- 2 091 976
GB-A- 1 500 080       US-A- 4 452 265
US-A1- 2007 117 997

## Beschreibung

Die Erfindung betrifft ein Verfahren zur Regelung der Viskosität eines mindestens zwei Komponenten unterschiedlicher Viskosität enthaltenden Gemischs, insbesondere von Monomeren und Oligomeren der Reihe der Diphenylmethandiisocyanaten (MDI).

Bei der Herstellung eines Produkts, welches sich aus mehreren Komponenten zusammensetzt, sind dessen Eigenschaften vom Mischungsverhältnis der Komponenten abhängig. Häufig entstehen solche Mischungen als Ergebnis einer oder mehrerer chemischer Reaktionen und das Mischungsverhältnis ist abhängig von den bei der Reaktion herrschenden Bedingungen. Trotz aller Anstrengungen die Bedingungen bei der Reaktion konstant zu halten, kommt es immer wieder zu Variationen mit der Folge, dass die Eigenschaften des erhaltenen Produkts den geforderten Eigenschaften nicht mehr entsprechen.

Beispielsweise entsteht bei der Synthese von Diaminodiphenylmethan (MDA) aus Anilin und Formaldehyd mittels saurer Katalyse ein Gemisch aus 3 verschiedenen Isomeren des Monomers (monomeres MDA) sowie verschiedener langkettiger Oligomere. Aus diesem Roh-MDA Gemisch wird durch Umsetzung des Roh-MDA mit Phosgen ein Roh-Diphenylmethandiisocyanate (Roh-MDI) enthaltendes Gemisch gewonnen. Auch das Roh-MDI ist ein Gemisch von 3 verschiedenen Isomeren des Monomers (monomeres MDI) mit verschiedenen langkettigen Oligomeren, wobei das Verhältnis der Komponenten zueinander im Wesentlichen dem des Roh-MDA entspricht. Aus dem Roh-MDI wird ein Gemisch aus Diphenylmethandiisocyanaten (MDI) gewonnen, dem ein Teil der im Ausgangsgemisch enthaltenen Monomere abgetrennt wird. Die verbleibende Restfraktion ist ein MDI Gemisch, welches das oligomere MDI und den Restanteil an monomerem MDI enthält, und wird oft als "Polymer-MDI" bezeichnet. Das Polymer-MDI (PMDI) Gemisch wird aus dieser Trennstufe in ein Tanklager abgeführt. Das gewonnene monomere MDI kann in einer weiteren sich anschließenden Trennstufe (Destillation oder Kristallisation) in verschiedene Monomer-MDI Produkte aufgetrennt werden.

Für die Eigenschaften des so erhaltenen PMDI Gemischs, insbesondere für dessen Viskosität, ist der Anteil an monomerem MDI im Gemisch entscheidend. Die Viskosität wird durch einen abnehmenden Anteil an monomerem MDI erhöht und verringert sich bei Erhöhung des monomeren MDI Anteils.

Zur Kontrolle der Produkteigenschaften, insbesondere der Viskosität, werden deswegen bei der Produktion regelmäßig Proben entnommen und anschließend im Labor auf ihre Eigenschaften untersucht. Werden Abweichungen von den Solleigenschaften festgestellt, werden die Parameter des Produktionsprozesses angepasst. Nachteilig an dieser Prozedur ist, dass zwischen der Probenentnahme und eventuell erforderlichen Anpassungen am Prozess eine erhebliche Zeitspanne liegt, in der der Produktionsprozess nicht geregelt werden kann. Sind die festgestellten Abweichungen in den Produkteigenschaften dabei zu groß, wird in dieser Zeit nur nicht verwertbarer Ausschuss produziert. Eine kontinuierliche Überwachung der Viskosität ist deswegen erforderlich, um gleichbleibende Produkteigenschaften garantieren zu können.

Aus der EP 1 480 033 A1 sind ein Verfahren und eine Vorrichtung zur Bestimmung der Isomerenzusammensetzung bei Isocyanat-Herstellprozessen bekannt. In dem offenbarten Verfahren wird die Zusammensetzung in einem Isocyanat-Isomerengemisch so bestimmt, dass beispielsweise online ein Spektrum des Isomerengemisches mit einem optischen Sensor mittels Nah-Infrarot-Spektroskopie, Mittelinfrarot-Spektroskopie oder Raman-Spektroskopie aufgenommen wird. Das gemessene Spektrum wird dann in ein chemometrisches Kalibrationsmodell eingegeben, das zuvor für die Mischung dieser Isomeren erstellt worden ist. Durch die Auswertung des Spektrums in dem chemometrischen Kalibrationsmodell erhält man die Isomerenkonzentrationen in dem Isomerengemisch. Durch Vergleich der so ermittelten Ist-Konzentration der Isomere mit spezifizierten Soll-Konzentrationen kann die Isomerentrennanlage entsprechend nachgeregelt werden.

Nachteilig an dem Verfahren ist, dass zu Beginn das chemometrische Modell durch häufige Probenentnahme und Analysen für jedes verwendete Isomerengemisch neu geeicht werden muss. Des Weiteren müssen auch im späteren Betrieb permanent Proben zur Optimierung dieses statistischen Modells entnommen und analysiert werden. Auch wenn im Durchschnitt nur eine geringe Standardabweichung resultiert, können im Einzelfall große absolute Abweichungen auftreten. Folglich ist die quantitative Zuverlässigkeit des Spektroskopie-Geräts nicht groß genug für eine wirksame Kontrolle des Prozesses. Die weiterhin erforderliche permanente Probenentnahme und die erforderlichen empfindlichen Sensoren bringen einen großen Wartungsaufwand sowie erhöhte Risiken und Aufwendungen bezüglich des Arbeits- und Umweltschutzes mit sich.

Aus der US 2004/0177679 A1 sind ein Rotationsviskosimeter und ein Verfahren zur Messung der Viskosität einer Flüssigkeit bekannt. Dabei wird gemessen, wie groß die Reibung zwischen der Flüssigkeit und einem rotierenden Bauteil ist. Das rotierende Teil wird mit einem Motor angetrieben und steht mit der Flüssigkeit in Kontakt, wobei ein Trennelement die mit der Flüssigkeit in Kontakt stehenden Teile vom Motor trennt. Dabei wird auf der Motorseite des Trennelements ein Druck aufgebaut, der über dem der Flüssigkeit liegt. Dadurch soll verhindert werden, dass Flüssigkeit in das Rotationsviskosimeter eindringt.

In CN 2881601 Y wird eine Vorrichtung zur Messung der Viskosität einer Flüssigkeit mit Temperaturkompensation offenbart. Die Messvorrichtung umfasst eine Stahlkugel, die sich in einer Glasröhre bewegt, wobei die Stahlkugel durch die zu testende Flüssigkeit gebremst wird. Die Flüssigkeit wird aus einer Hauptleitung über eine Bypass-Leitung entnommen und in die Messvorrichtung geleitet. Die Bewegung der Stahlkugel wird über Annäherungsschalter registriert. Zudem wird für eine Korrektur der gemessenen Werte die Temperatur gemessen.

Von Nachteil bei diesen und weiteren mechanischen Messverfahren ist, dass diese anfällig für Leckagen sind und/oder diese sehr aufwändig abgedichtet werden müssen. Des Weiteren sind die mechanischen Messelemente anfällig für Verstopfungen. Aus der EP 1 932 828 A2 wird ein Verfahren zur Steuerung der Herstellung von Diphenylmethandiisocyanaten offenbart, wobei die Viskosität gemessen wird.

Aufgabe der vorliegenden Erfindung ist es, ein Verfahren bereitzustellen, durch welches eine vorgegebene Soll-Viskosität eines mindestens zwei Komponenten mit unterschiedlicher Viskosität enthaltenen Gemisches sichergestellt werden kann.

Gelöst wird die Aufgabe durch ein Verfahren zur Regelung der Viskosität eines mindestens zwei Komponenten mit unterschiedlicher Viskosität gemäß dem unabhängigen Anspruch 1. Das Gemisch, dessen Viskosität geregelt werden soll, ist ein Gemisch von mindestens zwei Komponenten mit unterschiedlicher Viskosität, wobei die Eigenschaften des Gemischs, insbesondere dessen Viskosität, vom Mischungsverhältnis der Komponenten abhängig sind. Bei den Komponenten kann es sich um verschiedene Isomere und/oder verschiedene Oligomere einer chemischen Verbindung handeln. Das Gemisch kann das Ergebnis einer chemischen Reaktion sein, wobei die bei der Reaktion herrschenden Bedingungen Einfluss auf das Mischungsverhältnis der Komponenten haben. Das Gemisch kann auch das Produkt eines Verarbeitungsschrittes sein, durch das dem Gemisch Komponenten hinzugefügt oder entzogen werden. Des Weiteren kann sich das Mischungsverhältnis bei der Lagerung durch weiterhin ablaufende Reaktionen ändern.

Im ersten Schritt (a) des Verfahrens wird die Viskosität des Gemischs ermittelt. Dies erfolgt durch Ultraschallmessungen an oder in einer das Gemisch führenden Leitung oder in einem Behälter, der das Gemisch aufnimmt. Die verwendete Ultraschall-Messeinrichtung kann dabei direkt an eine das Gemisch führende Leitung angebracht werden, beispielsweise auf einem Flansch einer Rohrleitung, wobei eine Messsonde der Ultraschall-Messeinrichtung in das Gemisch hineinragt. Ebenso ist es möglich, über einen Bypass einen Teil des Gemischs aus der Leitung zu entnehmen und der Messeinrichtung zuzuführen. Bei der Messung der Viskosität des Gemischs in einem Behälter wird die Messeinrichtung bevorzugt an einer Wand des Behälters angebracht oder in diese integriert.

Im nachfolgenden Schritt (b) des Verfahrens wird der im ersten Schritt (a) ermittelte Messwert auf Standardbedingungen normiert. Die Standardbedingungen umfassen eine vorgegebene Standardtemperatur. Hierzu wird die Temperatur des mindestens zwei Komponenten enthaltenden Gemischs gemessen und zusammen mit einem Modell zur Normierung auf die Standardtemperatur genutzt. Als Standardtemperatur wird beispielsweise eine Temperatur im Bereich von 20°C bis 50°C gewählt. Dabei können bereits mit einem einfachen Modell, welches einen Proportionalitätsfaktor B und einen Temperaturkorrekturfaktor y vorsieht, gute Ergebnisse erzielt werden:

$$U_{25°C} = B^{-1} \cdot Q \cdot (T/25)^y \qquad (I)$$

Dabei ist $U_{25°C}$ die auf eine Temperatur von 25°C normierte Viskosität, Q die gemessene Viskosität und T die gemessene Temperatur. Der Proportionalitätsfaktor B und der Temperaturkorrekturfaktor y werden durch Analyse einiger Proben des Gemischs im Labor und anschließende Kurvenanpassung ermittelt.

Mit einer zusätzlichen Konstante kann das Modell in einer anderen Temperatureinheit, zum Beispiel in Kelvin verwendet werden:

$$U_{298K} = B^{-1} \cdot Q \cdot ((T+A)/273,14)^y \qquad (II)$$

Die Konstante A, der Proportionalitätsfaktor B und der Temperaturkorrekturfaktor y werden durch Analyse einiger Proben des Gemischs im Labor ermittelt.

Im dritten Verfahrensschritt (c) wird der auf Standardbedingungen normierte Viskositätsmesswert als Ist-Wert mit einem vorgegebenen Soll-Wert verglichen. Weichen Ist-und Soll-Wert voneinander ab, wird im letzten Schritt (d) des Verfahrens die Viskosität des Gemischs angepasst, indem das Mischungsverhältnis der einzelnen Komponenten zueinander geändert wird. Dies wird erreicht, indem der Anteil mindestens einer Komponente des Gemischs erhöht oder verringert wird.

Das aus mehreren Komponenten zusammengesetzte Gemisch wird in einer Ausführungsform des Verfahrens durch Destillation oder Kristallisation aus einem Roh-Gemisch gewonnen, wobei mindestens eine Komponente zumindest teilweise abgetrennt wird. Dabei wird die Viskosität des durch Destillation oder Kristallisation erhaltenen Gemischs mittels Ultraschallmessungen im Ablaufstrom ermittelt.

Das Anpassen der Viskosität im Schritt (d) des Verfahrens erfolgt dann beispielsweise durch Vergrößern oder Verkleinern der abgetrennten Menge der mindestens einen durch die Destillation oder Kristallisation abgetrennten Komponente. Eine weitere Variante zum Anpassen der Viskosität ist das Beimischen einer Teilmenge der mindestens einen durch die Destillation oder Kristallisation abgetrennten Komponente.

Sofern das Roh-Gemisch Ergebnis eines Herstellungsprozesses ist, kann zur Anpassung der Viskosität im Schritt (d) des Verfahrens die Zusammensetzung des Roh-Gemischs durch Ändern der Prozessparameter des Herstellungsprozesses geändert werden.

Dabei ist es je nach Ausführungsform des erfindungsgemäßen Verfahrens denkbar, neben den drei soeben beschriebenen Möglichkeiten zur Anpassung der Viskosität auch eine Kombination mehrerer dieser Möglichkeiten einzusetzen. Dabei ist es des Weiteren möglich, das Anpassen der Viskosität so durchzuführen, dass die Menge der mindestens einen abgetrennten Komponente oder die Menge des durch Destillation oder Kristallisation gewonnenen Gemischs konstant ist.

Ein Beispiel für ein mindestens zwei Komponenten mit unterschiedler Viskosität enthaltenes Gemisch ist Diisocyanatodiphenylmethan (MDI). Die Herstellung von MDI erfolgt in mehreren Stufen. In der ersten Stufe wird Diaminodiphenylmethan (MDA) aus Anilin und Formaldehyd mittels saurer Katalyse synthetisiert. Das so erhaltene Roh-MDA ist ein Gemisch aus 3 verschiedenen Isomeren des Monomers (monomeres MDA) sowie verschiedener langkettiger Oligomere.

In der zweiten Stufe des Herstellungsverfahrens wird Phosgen aus Kohlenmonoxid und Chlor synthetisiert, das in der dritten Stufe mit dem Roh-MDA aus der ersten Stufe in einem Lösungsmittel zu Roh-MDI umgesetzt wird. Als Lösungsmittel wird in der Regel Mono- oder Dichlorbenzol eingesetzt.

Auch das Roh-MDI ist ein Gemisch von 3 verschiedenen Isomeren des monomeren MDI mit verschiedenen langkettigen Oligomeren, wobei das Verhältnis der Komponenten zueinander im Wesentlichen dem des Roh-MDA entspricht. Aus dem Roh-MDI wird ein Gemisch aus Diphenylmethandiisocyanaten (MDI) gewonnen, indem ein Teil des im Ausgangsgemisch enthaltenen monomeren MDI mittels Destillation im Vakuum abgetrennt wird. Das verbleibende MDI-Gemisch, enthaltend das oligomere MDI und den Restanteil an monomerem MDI, wird "Polymer-MDI" (PMDI) genannt und wird aus dieser Trennstufe in einen Lagertank abgeführt. Das gewonnene monomere MDI wird in einer weiteren sich anschließenden Trennstufe, zum Beispiel Destillation oder Kristallisation, in verschiedene MDI-Produkte aufgetrennt. Die Hauptprodukte sind 4,4'-MDI mit einer Reinheit von ca. 98% und ein Gemisch aus 2,4'- und 4,4'-MDI mit einem Mischungsverhältnis von ungefähr 50:50, wobei letzteres und das nach der Destillation verbleibende PMDI Gemisch geringe Mengen an 2,2'-Isomer enthalten.

Für die Eigenschaften des so erhaltenen PMDI Gemischs, insbesondere für dessen Viskosität, ist der Anteil an monomerem MDI entscheidend. Die Viskosität wird durch einen abnehmenden Anteil an monomerem MDI erhöht und verringert sich bei Erhöhung des monomeren MDI Anteils.

Die Viskosität des PMDI Gemischs ist demnach abhängig davon, wie viel monomeres MDI bei der Destillation abgetrennt wird. Dies wiederum ist abhängig von den Prozessbedingungen sowie der ursprünglichen Zusammensetzung des Roh-MDI. Zudem kommt es bei der Lagerung von frischem MDI zu Nebenreaktionen, durch die der Anteil an monomerem MDI abnimmt und sich die Viskosität erhöht.

Wird das erfindungsgemäße Verfahren in einem Prozess eingesetzt, bei dem ein PMDI-Gemisch durch Abtrennen von monomerem MDI mittels Destillation aus einem Roh-MDI Gemisch gewonnen wird, wird die Viskosität bevorzugt durch Ultraschallmessungen am Ablaufstrom des Produkts ermittelt.

Mit Hilfe der ermittelten, auf Standardbedingungen normierten Viskositätsmesswerte, kann eine Regelung erfolgen, so dass der gemessene Ist-Wert an einen vorgegebenen Soll-Wert angenähert wird.

Eine solche Regelung kann durch Ändern der Prozessparameter bei der Destillation erfolgen, so dass in Abhängigkeit der ermittelten Viskosität die Menge des aus dem Roh-MDI Gemischs abgetrennten monomerem MDI geregelt wird. Dies kann beispielsweise durch Regelung der zugeführten Energie bzw. Wärme erfolgen.

Die Regelung kann auch dadurch erfolgen, dass ein Teil des durch Destillation abgetrennten monomeren MDI dem PMDI-Gemisch wieder beigemischt wird, wobei die beizumischende Menge abhängig von der gemessenen und normierten Ist-Viskosität und der vorgegebenen Soll-Viskosität des MDI-Gemischs ist.

Des Weiteren kann in Abhängigkeit der ermittelten Viskosität die Zusammensetzung des Roh-MDI Gemischs geregelt werden. Da die Isomeren-Zusammensetzung des Roh-MDI im Wesentlichen der des ursprünglichen Roh-MDA entspricht, kann dies durch eine Anpassung der Prozessbedingungen bei der MDA Synthese erzielt werden. Dabei lässt sich beispielsweise der Anteil der Isomeren im Roh-MDA Gemisch durch Ändern des Mengenverhältnisses von Anilin zu Formaldehyd und/oder der eingesetzten Menge sauren Katalysators beeinflussen.

Je nach Ausführungsform des erfindungsgemäßen Verfahrens kann einer der drei soeben genannten Regelungsansätze alleine, oder auch eine Kombination aus mehreren der beschriebenen Regelungsansätze verwendet werden. Wird dabei die Menge an zugeführtem Roh-MDA konstant gehalten und die Zusammensetzung des Roh-MDA Gemischs nicht verändert, variieren die Mengen an abgetrenntem monomerem MDI und verbleibendem PMDI-Gemisch. Durch zusätzliches Regeln der Roh-MDA Zusammensetzung und/oder der Menge an zugeführtem Roh-MDA, kann zusätzlich zur Viskosität des PMDI-Gemischs auch das Verhältnis zwischen dem produzierten PMDI-Gemisch und der abgetrennten Menge monomeren MDI beeinflusst werden.

Ein weiteres Anwendungsbeispiel für das erfindungsgemäße Verfahren ergibt sich aus der Tatsache, dass die Viskosität von frisch produziertem MDI-Gemisch über die Zeit der Lagerung ansteigt. Ursache für diesen Effekt sind in dem Gemisch ablaufende Nebenreaktionen, die zur Bildung von längerkettigen Oligomeren mit höherer Viskosität führen. Dies hat jedoch zur Folge, dass das gelagerte Gemisch nicht mehr in jedem Fall die geforderten Eigenschaften aufweist.

Erfindungsgemäß wird vorgeschlagen, die Viskosität eines mindestens zwei Komponenten mit unterschiedlicher Viskosität enthaltenen Gemischs durch Änderung von dessen Zusammensetzung zu regeln, wobei die Messung der Viskosität des Gemischs an einer Leitung zwischen einem Behälter, der das aus mindestens zwei Komponenten zusammengesetzte Gemisch enthält, und einer Abfülleinrichtung durchgeführt wird.

Abhängig von der ermittelten normierten Viskosität des Gemischs kann dann die Zusammensetzung des im Behälter aufbewahrten Gemischs geändert werden. Dies kann beispielsweise durch die Zufuhr von einem weiteren Gemisch bewerkstelligt werden, wobei bei zu geringer Viskosität des Gemischs im Behälter ein Gemisch mit höherer Viskosität als das im Lagertank aufbewahrte Gemisch zugegeben wird und bei zu hoher Viskosität des Gemischs im Behälter ein Gemisch mit geringerer Viskosität als das im Behälter aufbewahrte Gemisch zugegeben wird.

Im Falle eines PMDI-Gemischs als mindestens zwei Komponenten mit unterschiedlicher Viskosität enthaltendes Gemisch wird frisch produziertes PMDI einem Behälter, in dem PMDI aufbewahrt wird, zugeführt, wobei die Viskosität des frisch produzierten Produkts gegebenenfalls durch Verändern des Soll-Werts für die Viskosität bei der Produktion beeinflusst wird. Dieser Soll-Wert wiederum wird so gewählt, dass die Viskosität des im Behälter gelagerten PMDI sich der beim Abfüllen gewünschten Viskosität annähert.

Wird das dem Behälter zugeführte PMDI-Gemisch durch Abtrennen von monomerem MDI mittels Destillation oder Kristallisation gewonnen, so kann das erfindungsgemäße Verfahren kaskadiert werden. Dabei wird in einer ersten Stufe nach einem der oben beschriebenen Verfahren die Viskosität eines frisch produzierten PMDI-Gemischs geregelt und mit diesem in einer zweiten Stufe die Viskosität eines in einem Behälter gelagerten PMDI-Gemischs eingestellt. Dabei werden die Ist- und Sollwerte der zweiten Stufe verwendet, um den Sollwert für die Viskosität in der ersten Stufe zu berechnen. Der Sollwert der zweiten Stufe ist die an der Abfülleinrichtung geforderte Viskosität, der Istwert der Viskosität der zweiten Stufe wird an der Verbindungsleitung zwischen dem Behälter und der Abfülleinrichtung gemessen. Der Istwert der ersten Stufe ist die an einer Verbindungsleitung zwischen der Einrichtung, in der das frische PMDI-Gemisch gewonnen wird, und dem Behälter gemessene Viskosität. Der Sollwert der ersten Stufe ist der Sollwert für die Viskosität des dem Behälter zugeführten frischen PMDI-Gemischs und wird demnach in Abhängigkeit der an der Verbindungsleitung zwischen dem Behälter und der Abfülleinrichtung gemessenen Ist-Viskosität eingestellt. Des Weiteren geht in die Berechnung des Sollwerts der ersten Stufe auch die für das abgefüllte PMDI-Gemisch geforderte Soll-Viskosität ein. Durch diese Verkettung der Viskositätsregelungen von Produktion und Lagerung wird ein frisches PMDI-Gemisch erzeugt, dessen Ist-Viskosität geeignet ist die Viskosität des im Behälter gelagerten PMDI-Gemischs der an der Abfülleinrichtung geforderten Soll-Viskosität anzunähern.

In einer Ausführungsform des erfindungsgemäßen Verfahrens wird das mindestens zwei Komponenten mit unterschiedlicher Viskosität enthaltende Gemisch in mindestens zwei Behältern aufbewahrt, wobei das Mischungsverhältnis der Komponenten des Gemischs zueinander in den jeweiligen Behältern unterschiedlich ist. Dabei ist vorgesehen, das Gemisch aus den Behältern über eine Abfülleinrichtung abzufüllen, das Gemisch aus den einzelnen Behältern vor dem Abfüllen in einer Mischeinrichtung zu vermischen und die Viskosität der Mischung über Ultraschallmessungen an einer Verbindungsleitung zwischen der Mischeinrichtung und der Abfülleinrichtung zu ermitteln. Die Auswahl des Mischungsverhältnisses bestimmt dann die Viskosität der abgefüllten Mischung. Die Mischeinrichtung kann ein statischer Mischer, ein weiterer Behälter oder ein Lagertank sein, der über entsprechende Rohrleitungen mit den einzelnen Behältern und der Abfüllanlage in Verbindung steht. Neben der Messung der Viskosität der Mischung können über zusätzliche Messeinrichtungen auch die Viskositäten der in den verschiedenen Behältern gelagerten Gemische gemessen werden. Diese zusätzlichen Messeinrichtungen sind beispielsweise direkt in den Behältern oder an den entsprechenden Verbindungsleitungen angeordnet.

Da im Falle von PMDI als mindestens zwei Komponenten mit unterschiedlicher Viskosität enthaltendes Gemisch die fortschreitende Erhöhung der Viskosität aus einer Abnahme des Gehalts an monomerem MDI resultiert, wird in einer Ausführungsform des erfindungsgemäßen Verfahrens zusätzlich zu dem gelagerten PMDI in mindestens einem weiteren Behälter monomeres MDI aufbewahrt. Dadurch kann in Abhängigkeit der gemessenen Gemischviskosität vor dem Abfüllen des PMDI in einer Mischeinrichtung monomeres MDI beigemischt werden. Die Viskositätsmessung erfolgt bevorzugt mittels Ultraschall an einer Verbindungsleitung zur Abfüllanlage.

Als Mischeinrichtung dient in den oben beschriebenen Fällen beispielsweise ein weiterer Lagertank oder ein statischer Mischer wie er beispielsweise in WO 2005 030841 beschrieben ist.

Im Folgenden wird die Erfindung anhand der Zeichnungen und der folgenden Beschreibung näher beschrieben. Darin zeigen:

Figur 1:    Eine schematische Darstellung der Viskositätsregelung bei der Trennung von Isomeren,

Figur 2:    die Normierung von Ultraschall-Viskositätsmessungen auf Standardbedingungen,

Figur 3:    eine schematische Darstellung der Viskositätsregelung bei der Mischung oder Lagerung von Isomeren.

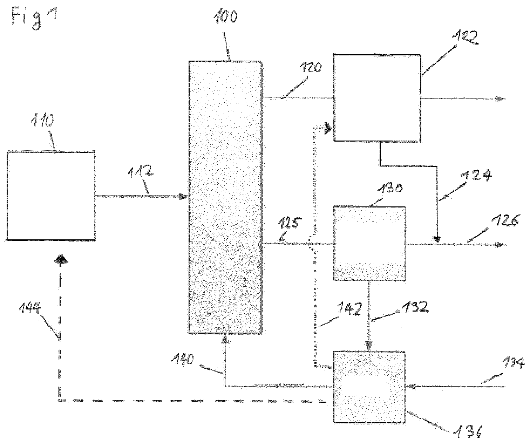

In Figur 1 ist das Regelschema einer Isomerentrennung mit Viskositätsregelung dargestellt.

Das Rohmaterial 112 wird in eine Trennvorrichtung 100 eingegeben, in der mindestens eine Komponente 120 ganz oder teilweise abgetrennt wird. Das verbleibende Gemisch 125 fließt aus der Trennvorrichtung 100 heraus zu einem Ablauf 126. Die Viskosität des verbleibenden Gemischs 125 ist abhängig davon, wie viel der mindestens einen Komponente 120 noch in dem Gemisch 125 enthalten ist. Zwischen der Trennvorrichtung 100 und dem Ablauf 126 ist eine Viskositäts-Messvorrichtung 130 angeordnet. Die Messvorrichtung 130 bestimmt die Viskosität des Gemischs 125 mit Hilfe von Ultraschall. Die gemessene Viskosität wird von der Messvorrichtung 130 auf Standardbedingungen normiert. Für die Normierung werden die Temperatur sowie gegebenenfalls der Massenstrom des Gemischs 125 berücksichtigt. Die normierte Viskosität wird als Ist-Viskosität 132 in einen Regler 136 eingegeben. Im Regler 136 wird die Ist-Viskosität mit einer vorgegebenen Soll-Viskosität 134 verglichen. Bei Abweichung der Ist- von der Soll-Viskosität werden je nach Ausführungsform ein oder mehrere Steuersignale 140, 142, 144 generiert. Über das Steuersignal 140 werden Prozessparameter der Trennvorrichtung 100 beeinflusst und somit die abgetrennte Menge der mindestens einen Komponente 120 gesteuert. Durch das Steuersignal 142 wird ein Regelventil 122 angesteuert, über das ein Teil der abgetrennten mindestens einen Komponente 120 dem Gemisch 125 beigemischt werden kann. Wird ein Gemisch aus mindestens zwei Komponenten 120 abgetrennt, welches in einem nachfolgenden Prozessschritt weiter aufgetrennt wird, können auch Teile einer oder mehrerer der aufgetrennten Komponenten 120 dem Gemisch 125 beigemischt werden. Mit dem Steuersignal 144 werden die Prozessparameter bei der Rohmaterialherstellung 110 beeinflusst, wodurch sich dessen Zusammensetzung ändert. Ebenfalls möglich ist es, mit dem Steuersignal 144 die Menge an zugeführten Rohmaterial 112 zu steuern.

Im Fall der PMDI Herstellung ist die Rohmaterialherstellung 110 die Synthese des Roh-MDI und die Trennvorrichtung 100 ist beispielsweise als Destillationsvorrichtung ausgeführt.

Wird das erfindungsgemäße Verfahren in einem Prozess eingesetzt, bei dem das mindestens zwei Komponenten mit unterschiedlichen Viskositäten enthaltende Gemisch ein Gemisch ist, das durch Abtrennen von monomerem MDI mittels Destillation aus einem Roh-MDI Gemisch gewonnen wird, wird die Viskosität bevorzugt durch Ultraschallmessungen am Ablaufstrom des Produkts ermittelt. Dabei ist das monomere MDI die abgetrennte Komponente 120 und besteht aus einem Gemisch der verschiedenen Isomere des monomeren MDI.

Die Viskosität des PMDI-Gemischs wird in der zum Ablauf 126 führenden Leitung mit einer als Ultraschall-Messvorrichtung ausgebildeten Viskositäts-Messvorrichtung 130 ermittelt, wobei in der Viskositäts-Messvorrichtung 130 auch eine Temperaturkompensation sowie gegebenenfalls eine Kompensation für den Massestrom erfolgt. Mit Hilfe der ermittelten, auf Standardbedingungen normierten Viskositätsmesswerte 132, kann nun über den Regler 136 eine Regelung erfolgen, so dass die gemessene Ist-Viskosität 132 an eine vorgegebene Soll-Viskosität 134 angenähert wird.

Eine solche Regelung kann über das Regelsignal 140 durch Ändern der Prozessparameter bei der Destillation 100 erfolgen, so dass in Abhängigkeit der ermittelten Viskosität 132 die Menge des aus dem Roh-MDI Gemisch 112 abgetrennten monomeren MDI-Gemischs 120 geregelt wird. Dies kann beispielsweise durch Regelung der zugeführten Wärme bzw. Energie erfolgen.

Die Regelung kann auch dadurch erfolgen, dass über ein Ventil 122 ein Teil des durch Destillation abgetrennten monomeren MDI 120 dem PMDI-Gemisch 125 wieder beigemischt wird, wobei die beizumischende Menge abhängig von der gemessenen und normierten Ist-Viskosität 132 und der vorgegebenen Soll-Viskosität 134 des PMDI-Gemischs ist und über das Regelsignal 142 an das Ventil 122 übermittelt wird. Nach demselben Prinzip kann auch ein Teil einer oder mehrerer Komponenten des in einem folgenden Prozessschritt noch weiter aufgetrennten monomeren MDI-Gemischs 120 dem PMDI-Gemisch 125 zugeführt werden.

Des Weiteren kann über das Steuersignal 144 in Abhängigkeit der ermittelten Ist-Viskosität 132 die Menge und Zusammensetzung des Roh-MDI Gemischs 112 geregelt werden. Da die Isomeren-Zusammensetzung des Roh-MDI Gemischs 112 im Wesentlichen der des als Zwischenprodukt anfallenden Roh-MDA entspricht, kann dies durch eine Anpassung der Prozessbedingungen bei der MDA Synthese erzielt werden. Dabei lässt sich beispielsweise der Anteil an Monomeren im Roh-MDA-Gemisch durch Ändern des Mengenverhältnisses von Anilin zu Formaldehyd und/oder der eingesetzten Menge sauren Katalysators beeinflussen.

Je nach Ausführungsform des erfindungsgemäßen Verfahrens kann eine der drei soeben genannten Regelungsansätze alleine, oder auch eine Kombination aus mehreren der beschriebenen Regelungsansätze verwendet werden.

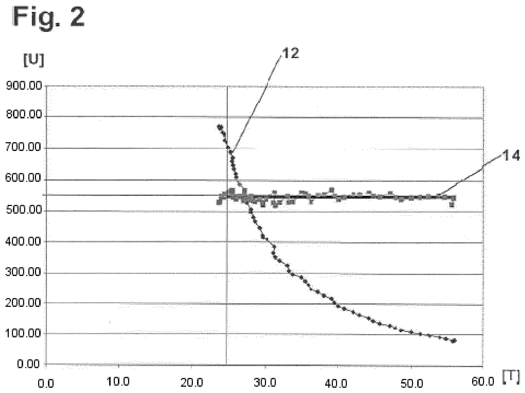

In der Figur 2 ist eine Messung der Viskosität von MDI in Abhängigkeit der Temperatur mit und ohne Normierung dargestellt.

In dem in Figur 2 dargestellten Diagramm ist die Viskosität einer MDI Probe gegen die Temperatur aufgetragen. Dabei zeigt die Y-Achse die Viskosität [U] und auf der X-Achse ist die Temperatur [T] aufgetragen, wobei die X-Achse einen Temperaturbereich von 0°C bis 60°C umfasst. Die Kurve 12 zeigt dabei die gemessene Viskosität der Probe. Der Messpunkt bei einer Temperatur von 25°C wird verwendet, um die Viskosität der Probe auf Standardbedingungen zu normieren. Das Ergebnis der Normierung ist als Kurve 14 im Diagramm dargestellt.

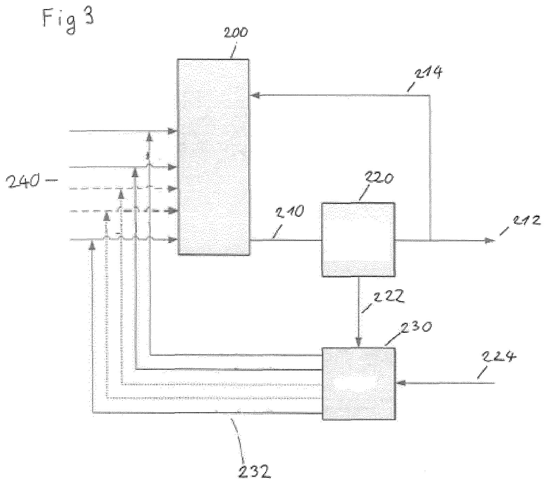

In Figur 3 ist das Regelschema zur Viskositätsregelung bei der Lagerung bzw. Abfüllung eines mindestens zwei Komponenten mit unterschiedlichen Viskositäten enthaltenden Gemischs dargestellt.

Bevor das mindestens zwei Komponenten mit unterschiedlichen Viskositäten enthaltende Gemisch über die Abfüllleitung 212 abgefüllt wird, wird dessen Viskosität mit einer Ultraschall-Messvorrichtung 220 ermittelt. Die gemessene Viskosität des Gemischs wird von der Messvorrichtung 220 auf Standardbedingungen normiert. Die auf Standardbedingungen normierte Ist-Viskosität 222 wird in einem Regler 230 mit einer vorgegebenen Soll-Viskosität 224 verglichen. Bei Abweichungen der Ist-Viskosität 222 von der Soll-Viskosität 224 werden ein oder mehrere Regelsignale 232 generiert, mit denen die Zufuhr von einem oder mehreren Gemischen 240 in die Mischvorrichtung 200 gesteuert wird.

Im Falle von PMDI als mindestens zwei Komponenten mit unterschiedlichen Viskositäten enthaltendem Gemisch kann der Regler 230 abhängig von der Abweichung der Ist-Viskosität 222 von der Soll-Viskosität 224 über ein Regelsignal 232 die Zufuhr von frisch produziertem PMDI in eine als Tank ausgeführte Mischvorrichtung 200 regeln. Dabei kann gegebenenfalls die Viskosität des frisch produzierten PMDI durch Verändern des Soll-Werts für die Viskosität bei der Produktion beeinflusst werden.

Stehen mehrere verschiedene MDI Gemische mit unterschiedlichen Viskositäten zur Verfügung, kann über den Regler 230 das Verhältnis, in dem die verschiedenen MDI-Gemische der Mischvorrichtung 200 zugeführt werden, eingestellt werden. Da in einem PMDI-Gemisch die fortschreitende Erhöhung der Viskosität aus einer Abnahme des Gehalts an monomerem MDI resultiert, wird in einer Ausführungsform des erfindungsgemäßen Verfahrens zusätzlich zu dem gelagerten PMDI-Gemisch in mindestens einem weiteren Lagertank monomeres MDI aufbewahrt. Dadurch kann in Abhängigkeit der gemessenen Ist-Viskosität 222 des Gemischs über das Regelsignal 232 vor dem Abfüllen des PMDI-Gemischs in einer Mischeinrichtung 200 monomeres MDI beigemischt werden.

In einer Variante des Verfahrens kann die Viskosität des mindestens zwei Komponenten mit unterschiedlichen Viskositäten enthaltenden Gemischs in einer als Tank ausgeführten Mischvorrichtung 200 ermittelt werden, ohne das Gemisch abzufüllen. Dazu wird das Gemisch über die Leitung 210 an der Ultraschall-Messvorrichtung 220 vorbeigeführt, und gelangt über die Rückführleitung 214 zurück in den Tank 200.

Bezugszeichenliste

[U]     Viskosität
[T]     Temperatur
12      Viskosität
14      normierte Viskosität
100     Trennvorrichtung
110     Roh-MDI Produktion
112     Rohmaterial
120     abgetrennte Komponente (monomeres MDI)
122     Ventil
124     zurückgeführtes 2 Kern-MDI
125     verbleibendes Gemisch
126     Ablauf (PMDI-Gemisch)
130     Viskositätsmessvorrichtung
132     Ist-Viskosität
134     Soll-Viskosität
136     Regler
140     Steuersignal (Destillation/Kristallisation) Trennung
142     Steuersignal Rückführung
144     Steuersignal MDI Produktion
200     Mischvorrichtung
212     Abfüllleitung
214     Rückführleitung
220     Messvorrichtung
222     Ist-Viskosität

224    Soll-Viskosität
230    Regler
232    Regelsignal
240    Gemisch

**Patentansprüche**

1. Verfahren zur Regelung der Viskosität eines mindestens zwei Komponenten mit unterschiedlicher Viskosität enthaltenden Gemischs (125; 210), folgende Schritte umfassend:

    (a) Ermitteln der Viskosität des Gemischs (125; 210) mittels Ultraschallmessungen,
    (b) Normieren der ermittelten Viskosität (132; 222) auf Standardbedingungen,
    (c) Vergleichen der normierten Viskosität mit einem vorgegeben Sollwert (134; 224),
    (d) Anpassen der Viskosität des Gemischs (125; 210) durch Erhöhen oder Verringern des Anteils mindestens einer Komponente des Gemischs,

wobei das mindestens zwei Komponenten mit unterschiedlicher Viskosität enthaltende Gemisch (125) monomere Diphenylmethandiisocyanate sowie oligomere Diphenylmethandiisocyanate enthält, wobei die Ultraschallmessungen gemäß Schritt (a) an oder in einer das Gemisch führenden Leitung oder in einem Behälter erfolgen, wobei, das aus mehreren Komponenten zusammengesetzte Gemisch (125) durch Destillation oder Kristallisation aus einem Roh- Diphenylmethandiisocyanate (MDI)-Gemisch (112) gewonnen wird, wobei mindestens eine Komponente zumindest teilweise abgetrennt wird und wobei das Roh-MDI-Gemisch (112) Ergebnis eines Herstellungsprozesses ist, bei dem das Roh-MDI-Gemisch (112) aus einem Roh- Diaminodiphenylmethan (MDA)-Gemisch durch Umsetzung mit Phosgen erzeugt wird, und zur Anpassung der Viskosität im Schritt (d) des Verfahrens die abgetrennte Menge der mindestens einen Komponente durch die Destillation oder Kristallisation vergrößert oder verkleinert wird und die Zusammensetzung des Roh-MDI-Gemischs (112) durch Ändern der Prozessparameter bei der MDA Synthese so geändert wird, dass die Menge der mindestens einen abgetrennten Komponente oder die Menge des durch Destillation oder Kristallisation gewonnenen Gemischs (125) konstant ist.

2. Verfahren nach Anspruch 1, **dadurch gekennzeichnet, dass** die Viskosität des durch Destillation oder Kristallisation erhaltenen Gemischs (125) mittels Ultraschallmessungen (130) im Ablaufstrom ermittelt wird.

3. Verfahren nach Anspruch 1 oder 2, **dadurch gekennzeichnet, dass** das Anpassen der Viskosität im Schritt (d) des Verfahrens durch Vergrößern oder Verkleinern der abgetrennten Menge von mindestens einer durch die Destillation oder Kristallisation abgetrennten Komponente durch Änderung der Energiezufuhr oder Energieabfuhr erfolgt.

4. Verfahren nach einem der Ansprüche 1 bis 3, **dadurch gekennzeichnet, dass** das Anpassen der Viskosität im Schritt (d) des Verfahrens durch Beimischen einer Teilmenge von mindestens einer durch die Destillation oder Kristallisation abgetrennten Komponente oder durch Beimischen eines Rohproduktgemischs mit einer anderen Zusammensetzung erfolgt.

5. Verfahren nach einem der Ansprüche 1 bis 4, **dadurch gekennzeichnet, dass** das mindestens zwei Komponenten mit unterschiedlicher Viskosität enthaltende Gemisch in einem Behälter (200) aufbewahrt wird und über eine Abfülleinrichtung abgefüllt werden kann und dass die Messung der Viskosität des Gemischs im Schritt (a) des Verfahrens an einer Verbindungsleitung (210) zwischen dem Behälter und der Abfülleinrichtung vorgenommen wird.

6. Verfahren nach Anspruch 5, **dadurch gekennzeichnet, dass** bei zu geringer Viskosität des im Behälter (200) aufbewahrten Gemischs die Viskosität durch Zufuhr von einem Gemisch mit höherer Viskosität als dem im Behälter (200) aufbewahrtem Gemisch erhöht wird und dass bei einer zu hohen Viskosität des im Behälter (200) aufbewahrten Gemischs die Viskosität durch Zufuhr von einem Gemisch mit niedrigerer Viskosität als dem im Behälter (200) aufbewahrten Gemisch verringert wird.

7. Verfahren nach Anspruch 6, **dadurch gekennzeichnet, dass** zwei Regelungen kaskadiert werden, wobei in einer ersten Stufe die Viskosität des dem Behälter (200) zugeführten Gemischs über ein Verfahren nach einem der Ansprüche 1 bis 6 geregelt wird und mit diesem frischen Gemisch in einer zweiten Stufe die Viskosität des in dem Behälter (200) aufbewahrten Gemischs eingestellt wird, wobei in die Berechnung des Sollwerts (134) der ersten Stufe die an der Verbindungsleitung (210) zwischen dem Behälter und der Abfülleinrichtung gemessene Viskosität und der Sollwert der Viskosität der zweiten Stufe berücksichtigt werden.

8.  Verfahren nach einem der Ansprüche 1 bis 4, **dadurch gekennzeichnet, dass** das mindestens zwei Komponenten mit unterschiedlicher Viskosität enthaltende Gemisch in mindestens zwei Behältern aufbewahrt wird und über eine Abfülleinrichtung abgefüllt werden kann, wobei das Mischungsverhältnis der Komponenten des Gemischs zueinander in den jeweiligen Behältern unterschiedlich ist, und dass das Gemisch aus den einzelnen Behältern vor dem Abfüllen in einer Mischeinrichtung vermischt wird und die Viskosität der Mischung über Ultraschallmessungen an einer Verbindungsleitung zwischen der Mischeinrichtung und der Abfülleinrichtung ermittelt wird, wobei über die Auswahl des Mischungsverhältnis die Viskosität der abgefüllten Mischung eingestellt wird.

9.  Verfahren nach Anspruch 8, wobei als Mischeinrichtung (200) ein weiterer Behälter oder ein statischer Mischer verwendet wird.

10.  Verfahren nach einem der Ansprüche 1 bis 4, **dadurch gekennzeichnet, dass** bei der Destillation oder Kristallisation die monomoren Diphenylmethandiisocyanate zumindest teilweise aus dem Gemisch abgetrennt werden.

11.  Verfahren nach einem der vorherigen Ansprüche, **dadurch gekennzeichnet, dass** die Ultraschallmessung gemäß Schritt (a) mittels Messen der Abstrahlverluste einer im von dem Gemisch umgebenen Ultraschallsonde erfolgt.

12.  Verfahren nach einem der vorherigen Ansprüche, **dadurch gekennzeichnet, dass** bei der Normierung der ermittelten Viskosität auf Standardbedingungen im Schritt (b) des Verfahrens Messabweichungen aufgrund einer von den Standardbedingungen abweichenden Gemischtemperatur kompensiert werden.

**Claims**

1.  A method of regulating the viscosity of a mixture (125; 210) comprising at least two components having different viscosities, which comprises the steps:

    (a) determination of the viscosity of the mixture (125; 210) by means of ultrasound measurements,
    (b) standardization of the viscosity determined (132; 222) to standard conditions,
    (c) comparison of the standardized viscosity with a prescribed intended value (134; 224),
    (d) adjustment of the viscosity of the mixture (125; 210) by increasing or decreasing the proportion of at least one component of the mixture,

    where the mixture (125) comprising at least two components having different viscosities comprises monomeric diphenylmethane diisocyanates and oligomeric diphenylmethane diisocyanates, where the ultrasound measurements in step (a) are carried out at or in a line conveying the mixture or in a vessel, where the mixture (125) composed of a plurality of components is obtained from a crude diphenylmethane diisocyanates (MDI) mixture (112) by distillation or crystallization, with at least one component being at least partly separated off and where the crude MDI mixture (112) is the result of a production process in which the crude MDI mixture (112) is produced from a crude diaminodiphenylmethane (MDA) mixture by reaction with phosgene, and in order to adjust the viscosity in step (d) of the method, the amount separated off of the at least one component is increased or decreased by the distillation or crystallization and the composition of the crude MDI mixture (112) is changed by changing the process parameters in the MDA synthesis in such a way that the amount of the at least one component separated off or the amount of the mixture (125) obtained by distillation or crystallization is constant.

2.  The method according to claim 1, wherein the viscosity of the mixture (125) obtained by distillation or crystallization is determined by means of ultrasound measurements (130) in the outflow stream.

3.  The method according to claim 1 or 2, wherein the adjustment of the viscosity in step (d) of the method is effected by increasing or decreasing the amount separated off of at least one component separated off by distillation or crystallization by changing the introduction of energy or removal of energy.

4.  The method according to any of claims 1 to 3, wherein the adjustment of the viscosity in step (d) of the method is effected by mixing in a partial amount of at least one component separated off by distillation or crystallization or by mixing in a crude product mixture having a different composition.

**5.** The method according to any of claims 1 to 4, wherein the mixture comprising at least two components having different viscosities is stored in a vessel (200) and can be dispensed via a dispensing device and the measurement of the viscosity of the mixture in step (a) of the method is carried out at a connecting line (210) between the vessel and the dispensing device.

**6.** The method according to claim 5, wherein, when the viscosity of the mixture stored in the vessel (200) is too low, the viscosity is increased by introducing a mixture having a higher viscosity than the mixture stored in the vessel (200) and when the viscosity of the mixture stored in the vessel (200) is too high, the viscosity is reduced by introducing a mixture having a lower viscosity than the mixture stored in the vessel (200).

**7.** The method according to claim 6, wherein two regulations are cascaded, with the viscosity of the mixture fed to the vessel (200) being regulated in a first stage by a method according to any of claims 1 to 6 and the viscosity of the mixture stored in the vessel (200) being set in a second stage by means of this fresh mixture, with the viscosity measured at the connecting line (210) between the vessel and the dispensing device and the intended value of the viscosity in the second stage being taken into account in the calculation of the intended value (134) in the first stage.

**8.** The method according to any of claims 1 to 4, wherein the mixture comprising at least two components having different viscosities is stored in at least two vessels and can be dispensed via a dispensing device, with the mixing ratio of the components of the mixture to one another being different in the respective vessels, and the mixture from the individual vessels is mixed in a mixing device before dispensing and the viscosity of the mixture is determined by ultrasound measurements at a connecting line between the mixing device and the dispensing device, with the viscosity of the dispensed mixture being set by selection of the mixing ratio.

**9.** The method according to claim 8, wherein a further vessel or a static mixer is used as mixing device (200).

**10.** The method according to any of claims 1 to 4, wherein the monomeric diphenylmethane diisocyanates are at least partly separated off from the mixture in the distillation or crystallization.

**11.** The method according to any of the preceding claims, wherein the ultrasound measurement in step (a) is carried out by measurement of the radiation losses of an ultrasonic probe surrounded by the mixture.

**12.** The method according to any of the preceding claims, wherein measurement deviations due to a mixture temperature deviating from the standard conditions are compensated in the standardization of the viscosity determined to standard conditions in step (b) of the method.

**Revendications**

**1.** Procédé de régulation de la viscosité d'un mélange (125 ; 210) contenant au moins deux composants de viscosité différente, comprenant les étapes suivantes :

(a) la détermination de la viscosité du mélange (125 ; 210) au moyen de mesures à ultrasons,
(b) la normalisation de la viscosité mesurée (132 ; 222) aux conditions standards,
(c) la comparaison de la viscosité normée à une valeur de consigne prédéfinie (134 ; 224),
(d) l'adaptation de la viscosité du mélange (125 ; 210) par élévation ou réduction de la proportion d'au moins un composant du mélange,

le mélange (125) contenant au moins deux composants de viscosité différente contenant des diisocyanates de diphénylméthane monomères, ainsi que des diisocyanates de diphénylméthane oligomères, les mesures à ultrasons selon l'étape (a) ayant lieu sur ou dans une conduite acheminant le mélange ou dans un contenant, le mélange (125) composé par plusieurs composants étant obtenu par distillation ou cristallisation d'un mélange de diisocyanates de diphénylméthane (MDI) brut (112), au moins un composant étant au moins partiellement séparé et le mélange de MDI brut (112) étant le résultat d'un procédé de fabrication selon lequel le mélange de MDI brut (112) est formé à partir d'un mélange de diaminodiphénylméthane (MDA) brut par réaction avec du phosgène, et, pour l'adaptation de la viscosité à l'étape (d) du procédé, la quantité séparée dudit au moins un composant par la distillation ou la cristallisation étant augmentée ou réduite, et la composition du mélange de MDI brut (112) étant modifiée par modification des paramètres du procédé lors de la synthèse de MDA, de telle sorte que la quantité dudit au moins un composant séparé ou la quantité du mélange (125) obtenu par distillation ou cristallisation soit constante.

**2.** Procédé selon la revendication 1, **caractérisé en ce que** la viscosité du mélange (125) obtenu par distillation ou cristallisation est déterminée par des mesures à ultrasons (130) dans le courant de sortie.

**3.** Procédé selon la revendication 1 ou 2, **caractérisé en ce que** l'adaptation de la viscosité à l'étape (d) du procédé a lieu par augmentation ou réduction de la quantité séparée d'au moins un composant séparé par distillation ou cristallisation par modification de l'apport d'énergie ou de la dissipation d'énergie.

**4.** Procédé selon l'une quelconque des revendications 1 à 3, **caractérisé en ce que** l'adaptation de la viscosité à l'étape (d) du procédé a lieu par incorporation d'une partie d'au moins un composant séparé par la distillation ou cristallisation ou par incorporation d'un mélange de produits brut ayant une autre composition.

**5.** Procédé selon l'une quelconque des revendications 1 à 4, **caractérisé en ce que** le mélange contenant au moins deux composants de viscosité différente est stocké dans un contenant (200) et peut être mis en bouteilles par un dispositif de mise en bouteilles, et **en ce que** la mesure de la viscosité du mélange à l'étape (a) du procédé est réalisée au niveau d'une conduite de raccordement (210) entre le contenant et le dispositif de mise en bouteilles.

**6.** Procédé selon la revendication 5, **caractérisé en ce qu'**en cas de viscosité trop faible du mélange stocké dans le contenant (200), la viscosité est augmentée par introduction d'un mélange de viscosité plus élevée que le mélange stocké dans le contenant (200), et **en ce qu'**en cas de viscosité trop élevée du mélange stocké dans le contenant (200), la viscosité est réduite par introduction d'un mélange de viscosité plus faible que le mélange stocké dans le contenant (200).

**7.** Procédé selon la revendication 6, **caractérisé en ce que** deux régulations sont réalisées en cascade ; lors d'une première étape, la viscosité du mélange introduit dans le contenant (200) étant régulée par un procédé selon l'une quelconque des revendications 1 à 6, et la viscosité du mélange stocké dans le contenant (200) étant ajustée avec ce mélange frais lors d'une deuxième étape, la viscosité mesurée au niveau de la conduite de raccordement (210) entre le contenant et le dispositif de mise en bouteilles et la valeur de consigne de la viscosité de la deuxième étape étant prises en compte lors du calcul de la valeur de consigne (134) de la première étape.

**8.** Procédé selon l'une quelconque des revendications 1 à 4, **caractérisé en ce que** le mélange contenant au moins deux composants de viscosité différente est stocké dans au moins deux contenants, et peut être mis en bouteilles par un dispositif de mise en bouteilles, le rapport de mélange entre les composants du mélange dans chaque contenant étant différent, et le mélange issu des contenants individuels étant mélangé dans un dispositif de mélange avant le mise en bouteilles, et la viscosité du mélange étant déterminée par des mesures à ultrasons au niveau d'une conduite de raccordement entre le dispositif de mélange et le dispositif de mise en bouteilles, le choix du rapport de mélange permettant d'ajuster la viscosité du mélange mis en bouteilles.

**9.** Procédé selon la revendication 8, dans lequel un contenant supplémentaire ou un mélangeur statique est utilisé en tant que dispositif de mélange (200).

**10.** Procédé selon l'une quelconque des revendications 1 à 4, **caractérisé en ce que** les diisocyanates de diphénylméthanes monomères sont au moins partiellement séparés du mélange lors de la distillation ou de la cristallisation.

**11.** Procédé selon l'une quelconque des revendications précédentes, **caractérisé en ce que** la mesure à ultrasons selon l'étape (a) a lieu par mesure des pertes de rayonnement d'une sonde à ultrasons entourée par le mélange.

**12.** Procédé selon l'une quelconque des revendications précédentes, **caractérisé en ce que**, lors de la normalisation de la viscosité déterminée aux conditions standards à l'étape (b) du procédé, des fluctuations de mesure dues à une température de mélange différente des conditions standards sont compensées.

Fig 1

## Fig. 2

Fig 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1480033 A1
- US 20040177679 A1
- CN 2881601 Y
- EP 1932828 A2
- WO 2005030841 A